# EUROPÄISCHE PATENTSCHRIFT

(11) **EP 0 406 714 B1**
(45) Veröffentlichungstag und Bekanntmachung des Hinweises auf die Patenterteilung: **12.05.1993**
(21) Anmeldenummer: 90112415.6
(22) Anmeldetag: 29.06.1990
(51) Int. Cl.: C07C 51/60, C07C 53/42

(54) **Verfahren zur Herstellung von Carbonsäurechloriden**
Method for the production of carboxylic acid chlorides
Procédé pour la fabrication de chlorures d'acides carboxyliques

(30) Priorität: 07.07.1989 DE 3922362
(43) Veröffentlichungstag der Anmeldung: 09.01.1991
(73) Patentinhaber: BASF Aktiengesellschaft, 67063 Ludwigshafen (DE)
(72) Erfinder: Decker, Martin, Dr., D-6700 Ludwigshafen (DE); Franzischka, Wolfgang, Dr., D-6710 Frankenthal (DE); Irnich, Rudolf, Dr., D-6719 Bobenheim (DE); Sauerwald, Manfred, Dr., D-6701 Meckenheim (DE)

(56) Entgegenhaltungen:
- EP-A- 0 031 504
- DE-A- 2 057 956
- US-A- 3 318 950

## Beschreibung

Diese Erfindung betrifft ein verfahren zur Herstellung von Carbonsäurechloriden durch Umsetzung von Carbonsäuren oder deren Anhydriden mit Kohlenoxidchlorid in Gegenwart von Diisopropylformamid.

Aus der BE-PS 620 385 und der DE-OS 2 057 956 ist bekannt, daß man Carbonsäuren mit Kohlenoxidchlorid in Gegenwart von Dialkylcarbonsäureamiden als Katalysatoren in die entsprechenden Carbonsäurechloride überführen kann. Bei der technischen Durchführung dieses Verfahrens gibt es jedoch Probleme, die in EP-PS 31 504 näher erläutert sind. Danach treten Schwierigkeiten insbesondere bei Formamiden mit niederen Alkylresten, wie Diethyl-, Di-n-propyl- oder Diisopropylformamid auf, die in Form ihrer Amidiniumchloride zu unlöslichen Ablagerungen neigen, während bei der Verwendung von Diisobutylformamid günstigere Ergebnisse erhalten werden. Dabei wird das Dialkylformamid in Mengen von 0,1 bis 0,4 Mol%, bezogen auf die Carbonsäuren, angewendet.

Es wurde nun überraschend gefunden, daß man bei der Herstellung von Carbonsäurechloriden durch Umsetzung von Carbonsäuren oder deren Anhydriden mit Kohlenoxidchlorid in Gegenwart von Diisopropylformamid als Katalysator die genannten Nachteile vermeidet, wenn man Diisopropylformamid in einer Menge von 0,001 bis 0,09 Mol%, bezogen auf die Carbonsäure bzw. das Anhydrid, einsetzt.

Weder bei der diskontinuierlichen noch bei der kontinuierlichen Arbeitsweise werden bei dem erfindungsgemäßen Verfahren störende Ablagerungen oder Verfärbungen bei der destillativen Aufarbeitung der Carbonsäurechloride beobachtet. Erstaunlicherweise entfaltet der Katalysator in sehr niedrigen Konzentrationen eine hervorragende Aktivität, so daß auch bei Unterschuß oder einem sehr geringen überschuß an Kohlenoxidchlorid noch eine hohe Reaktionsgeschwindigkeit und damit ein hoher Durchsatz bewirkt wird und das Verfahren mit geringem Energieaufwand durchgeführt werden kann. Vorzugsweise wendet man das Diisopropylformamid in Mengen von 0,005 bis 0,08, insbesondere von 0,01 bis 0,05 Mol% bezogen auf die Carbonsäuren bzw. Anhydride an.

Bevorzugte Ausgangsstoffe sind aliphatische Carbonsäuren oder deren Anhydride. Beispielsweise geht man von Carbonsäuren der allgemeinen Formel R-COOH aus, in der R einen Alkylrest mit 1 bis 22 Kohlenstoffatomen bedeutet. Der Alkylrest kann noch unter den Reaktionsbedingungen inerte Substituenten wie Chloratome, Alkoxi- oder Alkoxicarbonylgruppen mit 1 bis 4 Kohlenstoffatomen enthalten. Folgende Ausgangsstoffe sind beispielsweise geeignet: Essigsäure, Mono-, Di-, Trichloressigsäure, Propionsäure, Buttersäure, Valeriansäure, Laurylsäure, Stearinsäure, Talgfettsäure, Behensäure, Capronsäure, Isovaleriansäure, Palmitinsäure, 2-Ethylhexansäure, Methoxiessigsäure.

Die aliphatische Carbonsäure wird mit der stöchiometrischen Menge oder zweckmäßig mit einem Überschuß an Kohlenoxidchlorid, vorzugsweise in einem Verhältnis von 1,1 bis 1,5 Mol Kohlenoxidchlorid je Mol Ausgangssäure, umgesetzt. Die Umsetzung wird bei Temperaturen von 30 bis 150°C, vorzugsweise 50 bis 100°C, drucklos oder unter Druck diskontinuierlich oder kontinuierlich durchgeführt.

Die diskontinuierliche Umsetzung erfolgt beispielsweise so, daß man die flüssige oder geschmolzene Carbonsäure in einem geeigneten Rührbehälter vorlegt, Diisopropylformamid darin löst und das Gemisch auf die gewünschte Reaktionstemperatur erwärmt. Bereits während des Aufheizens kann mit dem Einleiten von Kohlenoxidchlorid begonnen werden. Das Ende der Reaktion wird durch starken Kohlenoxidchlorid-Rückfluß und einen Abfall der Gasentwicklung angezeigt. Das Rohprodukt wird in der Regel destillativ aufgearbeitet, wobei der Vorlauf des Carbonsäurechlorids das im überschuß eingesetzte Kohlenoxidchlorid enthält, das beim nachfolgenden Ansatz wieder eingesetzt werden kann.

Bei der kontinuierlichen Arbeitsweise verfährt man z.B. so, daß man den Reaktor mit der Carbonsäure und dem Katalysator bis nahe an den überlauf füllt und bei der jeweiligen Reaktionstemperatur mit Kohlenoxidchlorid umsetzt. Anschließend werden dann Carbonsäure, Diisopropylformamid und Kohlenoxidchlorid gleichzeitig und kontinuierlich dem Reaktor zugeführt. Der stündliche Volumenstrom der Carbonsäure, der im allgemeinen 10 bis 40 % des Reaktorvolumens beträgt, kann, besonders bei höhermolekularen Carbonsäuren, auf 50 bis 80 % des Reaktorvolumens erhöht werden.

Diisopropylformamid wird kontinuierlich zugeführt. Es kann auch vorab in der Carbonsäure bzw. dem Anhydrid gelöst und mit dieser zusammen dem Reaktor zugeführt werden. Das Kohlenoxidchlorid kann bei der kontinuierlichen Durchführung in der Menge auf wenige Mol% über die stöchiometrische Menge begrenzt werden, weil in einer dem Reaktor nachgeschalteten Kolonne das überschüssige Kohlenoxidchlorid abgetrennt und sofort wieder dem Reaktor zugeführt wird.

Die nach dem erfindungsgemäßen Verfahren herstellbaren Carbonsäurechloride sind Lösungsmittel oder wertvolle Ausgangsstoffe für die Herstellung von Lösungsmitteln, Riechstoffen, Weichmachern, Waschmitteln, Peroxiden, Beizen und Pflanzenschutzmitteln.

### Beispiel 1

In einem Rührreaktor mit 2000 Volumenteilen Fassungsvermögen werden 682 Gewichtsteile Talgfettsäure und 0,032 Gewichtsteile (0,01 Mol%) 5 Diisopropylformamid vorgelegt, dann wird der Reaktorinhalt auf 75°C erhitzt. Bei dieser Temperatur werden 310 Gewichtsteile Kohlenoxidchlorid innerhalb von 3 h mit einem getauchten Rohr in die Reaktionsmischung eingeleitet. Nun wird noch 30 Minuten bei gleicher Temperatur unter Kohlenoxidchlorid-Rückfluß nachgerührt und dann überschüssiges Kohlenoxidchlorid mit Stickstoff ausgeblasen. Es werden 729 Gewichtsteile rohes Talgfettsäurechlorid erhalten. Die Ausbeute beträgt 99,8 Mol%.

### Beispiel 2

In einem mit Überlauf, Rückflußkühler und Zulaufeinrichtungen ausgestatteten Rührbehälter, dessen Fassungsvermögen bis zum Überlauf 300 Volumenteile beträgt, werden 144 Gewichtsteile 2-Ethylhexansäure und 0,064 Gewichtsteile (0,05 Mol%) Diisopropylformamid vorgelegt. Durch Einleiten von Kohlenoxidchlorid bei 75°C wird die 2-Ethylhexansäure vollständig zum Carbonsäurechlorid umgesetzt. Die Temperatur des Kühlmittels im Rückflußkühler beträgt -70°C. in das Reaktionsgemisch werden dann bei 75°C stündlich 184 Gewichtsteile 2-Ethylhexansäure, in der 0,082 Gewichtsteile (0,05 Mol%) Diisopropylformamid gelöst sind, und 140 Gewichtsteile Kohlendioxidchlorid eingeleitet. Aus dem Überlauf des Rührreaktors gelangt die rohe Reaktionslösung in einen Vorratsbehälter, aus dem sie der weiteren Verarbeitung zugeführt wird. Das rohe 2-Ethylhexansäurechlorid fällt in 99,9 %iger Reinheit an.

## Patentansprüche

1. Verfahren zur Herstellung von Carbonsäurechloriden durch Umsetzung von Carbonsäuren oder deren Anhydriden mit Kohlenoxidchlorid in Gegenwart von Diisopropylformamid, dadurch gekennzeichnet, daß man das Diisopropylformamid in einer Menge von 0,001 bis 0,09 Mol%, bezogen auf die Carbonsäure bzw. das Anhydrid, anwendet.

2. Verfahren nach Anspruch 1, dadurch gekennzeichnet, daß man das Diisopropylformamid in einer Menge von 0,005 bis 0,08 Mol%, bezogen auf die Carbonsäure bzw. das Anhydrid, anwendet.

3. Verfahren nach Anspruch 1, dadurch gekennzeichnet, daß man das Diisopropylformamid in einer Menge von 0,01 bis 0,05 Mol%, bezogen auf die Carbonsäure bzw. das Anhydrid, anwendet.

## Claims

1. A process for preparing carbonyl chlorides by reacting carboxylic acids or their anhydrides with carbon oxychloride in the presence of diisopropylformamide, wherein the diisopropylformamide is used in an amount of from 0.001 to 0.09 mol% based on the carboxylic acid or the anhydride.

2. A process as claimed in claim 1, wherein the diisopropylformamide is used in an amount of from 0.005 to 0.08 mol% based on the carboxylic acid or the anhydride.

3. A process as claimed in claim 1, wherein the diisopropylformamide is used in an amount of from 0.01 to 0.05 mol% based on the carboxylic acid or the anhydride.

## Revendications

1. Procédé de préparation de chlorures d'acides carboxyliques par réaction d'acides carboxyliques ou d'anhydrides de ceux-ci avec de l'oxychlorure de carbone en présence de diisopropylformamide, caractérisé en ce qu'on utilise le diisopropylformamide dans une proportion de 0,001 à 0,09% en moles, par rapport à l'acide carboxylique ou à l'anhydride.

2. Procédé selon la revendication 1, caractérisé en ce qu'on utilise le diisopropylformamide dans une proportion de 0,005 à 0,08% en moles, par rapport à l'acide carboxylique ou à l'anhydride.

3. Procédé selon la revendication 1, caractérisé en ce qu'on utilise le diisopropylformamide dans une proportion de 0,01 à 0,05% en moles, par rapport à l'acide carboxylique ou à l'anhydride.
